# FASCICULE DE BREVET EUROPEEN

(11) **EP 2 240 786 B1**
(45) Date de publication et mention de la délivrance du brevet: **25.05.2011**
(21) Numéro de dépôt: 09720036.4
(22) Date de dépôt: 27.01.2009
(51) Int. Cl.: G01N 35/02, G01N 35/04, G01N 33/49

(54) **ALIMENTATION D'UN APPAREIL AUTOMATIQUE D'ANALYSE EN CUVETTES DE RÉACTION**
ZUFUHR VON REAKTIONSKÜVETTEN IN EINE AUTOMATISCHE ANALYSEMASCHINE
REACTION CUVETTE SUPPLY TO AN AUTOMATIC ANALYSIS MACHINE

(30) Priorité: 05.02.2008 FR 0800585
(43) Date de publication de la demande: 20.10.2010
(73) Titulaire: Diagnostica Stago, 92600 Asnières (FR)
(72) Inventeur: GELIN, Jean-François, F -94000 Creteil (FR); BENIZRI, Julien, F-77360 Vaires Sur Marne (FR); CASAS, Dominique, F - 92250 La Garenne-colombes (FR)
(74) Mandataire: Ramey, Daniel
(86) Numéro de dépôt international: PCT/FR2009/000088
(87) Numéro de publication internationale: WO 2009/112692

(56) Documents cités:
- EP-A- 1 018 544
- WO-A-03/065048
- US-A1- 2004 179 923

## Description

L'invention concerne essentiellement un dispositif d'alimentation en cuvettes de réaction d'un appareil automatique d'analyse utilisable notamment pour la détermination des temps de modification de l'état physique d'un milieu et en particulier pour la détermination du temps de coagulation du sang.

On connaît, par le document EP-A-0325874, un procédé et un appareil d'analyse dans lesquels un échantillon de sang est déposé dans une cuvette contenant une bille ferromagnétique que l'on peut déplacer en mouvement périodique au moyen d'un champ magnétique extérieur. Les variations d'amplitude et/ou de fréquence des mouvements de la bille dans le temps, qui sont représentatives des changements de l'état physique du sang, sont détectées par des moyens appropriés.

Cet appareil peut être alimenté en cuvettes de la façon décrite dans les documents WO 03/065047 et WO 03/065048, dans lesquels des cuvettes sont solidarisées les unes aux autres pour former une rangée continue, au moyen d'un film souple fixé sur les cuvettes de façon à obturer au moins partiellement leurs ouvertures et empêcher les billes de sortir des cuvettes. La bande de cuvettes ainsi formée peut être enroulée dans une bobine, celle-ci comprenant par exemple un millier de cuvettes et pouvant être chargée sur un axe de rotation monté à cet effet dans un compartiment approprié de l'appareil, celui-ci comprenant des moyens pour dérouler pas à pas la bande de la bobine et faire passer les cuvettes une à une dans l'appareil.

Les opérateurs chargés du fonctionnement de l'appareil rencontrent parfois des difficultés lors des changements de bobine, car il faut successivement retirer la bobine vide, mettre en place une bobine pleine et introduire correctement dans l'appareil l'extrémité du film portant les cuvettes, pour que les moyens d'entraînement prévus dans l'appareil puissent faire défiler les cuvettes une à une dans l'appareil.

L'arrêt de l'appareil d'analyse est obligatoire à chaque changement de bobine et peut se prolonger si les opérateurs ont des difficultés à mettre en place une bobine pleine.

Il est par ailleurs coûteux de fixer un film sur les cuvettes pour les relier en une bande continue, d'autant que ce film est retiré des cuvettes lorsque celles-ci sont utilisées dans l'appareil d'analyse.

On connaît, par le document US 2004/179923 A1 un système d'alimentation d'un appareil d'analyse au moyen d'un magasin amovible contenant des cuvettes de réaction. Lorsque ce magasin est vide, une alarme est générée pour qu'un opérateur retire le magasin vide et le remplace par un magasin plein, l'appareil d'analyse étant arrêté pendant le changement de magasin.

On connaît aussi par le document EP 1 018 544 A1 un système de stockage de coupelles de réaction qui sont empilées verticalement entre des tiges de guidage sur un plateau comportant une poignée supérieure de transport. Les piles de coupelles sont utilisées l'une après l'autre dans un appareil d'analyse puis, lorsque le plateau est vide, une alarme est générée pour qu'un opérateur remplace le plateau vide par un plateau plein. L'appareil d'analyse est arrêté pendant le changement de plateau.

La présente invention a notamment pour but d'éviter ces inconvénients de la technique antérieure et d'apporter une solution simple, efficace et économique au problème de l'alimentation en cuvettes d'un appareil d'analyse du type décrit ci-dessus.

Elle propose à cet effet une cassette pour l'alimentation d'un appareil automatique d'analyse en cuvettes de réaction, caractérisée en ce qu'elle comprend :
- deux parois latérales parallèles assemblées fixement l'une à l'autre et comportant des moyens de support de rangées de cuvettes de réaction,
- une face d'extrémité fermée, qui est formée par des rebords des parois latérales et qui empêche les cuvettes de sortir de la cassette,
- et une face d'extrémité ouverte, opposée à la face d'extrémité fermée et équipée d'un obturateur coulissant, déplaçable entre une position de sortie des cuvettes et une position de retenue des cuvettes.

Les moyens de support des rangées de cuvettes comprennent des nervures formées en saillie sur les faces internes des parois latérales de la cassette et sur lesquelles reposent des rebords latéraux des cuvettes.

De plus, les faces internes des parois latérales de la cassette comportent des ailettes parallèles aux nervures de support des cuvettes et s'étendant au-dessus de celles-ci pour fermer au moins partiellement les extrémités supérieures ouvertes des cuvettes portées par ces nervures et empêcher la sortie de billes contenues dans les cuvettes.

La cassette comprend par ailleurs au moins un poussoir formé par une plaquette transversale qui s'étend entre les parois latérales et qui est guidée en translation sur les moyens de support des cuvettes, cette plaquette comportant des pattes ou oreilles latérales s'étendant à travers des fentes des parois latérales de la cassette pour former des moyens d'entraînement de la plaquette d'une extrémité à l'autre de la cassette afin de déplacer simultanément toutes les rangées de cuvettes vers l'extrémité ouverte de la cassette.

Une telle cassette peut contenir par exemple environ un millier de cuvettes de réaction, qui sont simplement disposées en rangées superposées dans la cassette et qui ne sont pas reliées les unes aux autres par un film ou par un moyen quelconque.

Le ou les poussoirs qui sont prévus dans la cassette et qui sont guidés en translation sur des moyens de support ou de guidage des cuvettes, permettent de déplacer toutes les cuvettes des rangées superposées simultanément vers l'extrémité ouverte de la cassette.

Cette cassette peut être réalisée de façon économique en métal ou en matériau plastique moulé par injection ou thermoformé.

L'invention propose également un appareil automatique d'analyse, en particulier pour la détermination des temps de modification de l'état physique d'un milieu, caractérisé en ce qu'il comprend :
- des moyens formant un logement de réception d'une cassette du type décrit ci-dessus,
- des moyens prévus à une extrémité de ce logement et formant une zone de stockage tampon de cuvettes,
- des premiers moyens de transfert pour déplacer les cuvettes de la cassette placée dans le logement jusque dans la zone de stockage tampon,
- et des seconds moyens de transfert pour déplacer les cuvettes de la zone de stockage tampon jusqu'à un point d'entrée des cuvettes dans l'appareil d'analyse.

Dans l'appareil selon l'invention, la zone de stockage tampon permet le fonctionnement continu de l'appareil quand on retire une cassette vide et qu'on la remplace par une cassette pleine. La quantité de cuvettes se trouvant dans la zone de stockage tampon est prévue pour assurer le fonctionnement de l'appareil pendant un laps de temps très supérieur à celui nécessité par un changement de cassette. Lorsqu'une nouvelle cassette pleine a été mise en place dans l'appareil et que la zone de stockage tampon est vide, elle est rechargée en cuvettes à partir de cette nouvelle cassette pendant que les seconds moyens de transfert assurent l'alimentation de l'appareil en cuvettes, comme on le verra plus en détail dans ce qui suit.

Par exemple, lorsque la cassette contient environ un millier de cuvettes réparties en dix huit rangées superposées, la zone de stockage tampon comprend dix huit rangées superposées de neuf cuvettes, chacune, ce qui permet un fonctionnement continu de l'appareil sur environ 30 minutes.

Selon une autre caractéristique de l'invention, les premiers moyens de transfert des cuvettes comprennent des moyens d'entraînement de la ou des plaquettes poussoir guidées en translation dans la cassette, ces moyens d'entraînement comprenant un chariot déplacé sur un rail de guidage longitudinal par des moyens moteurs et portant au moins une tige cylindrique transversale qui s'étend le long des rangées de cuvettes, perpendiculairement à ces rangées et qui portent des doigts destinés a coopérer avec les oreilles latérales de la ou des plaquettes poussoir pour les déplacer d'un bout à l'autre des rangées de cuvettes.

Cette tige cylindrique ou chaque tige cylindrique est montée pivotante autour de son axe sur le chariot, entre une position de travail où les doigts sont en prise avec les oreilles latérales d'une plaquette poussoir et une position de repos où les doigts sont écartés des oreilles latérales de cette plaquette poussoir.

Ainsi, lorsque les tiges cylindriques sont dans leur position de repos, elles peuvent être ramenées par le chariot dans une position initiale de départ en étant déplacées le long d'une cassette pleine qui vient d'être mise en place dans le logement et sont ensuite ramenées en position de travail pour venir en prise avec les oreilles latérales de la ou des plaques poussoir de cette cassette pleine.

La rotation de la ou des tiges cylindriques est contrôlée avantageusement par des cames portées par la ou par chaque tige cylindrique et qui coopèrent avec des butées prévues dans le logement de la cassette.

Le chariot portant cette ou ces tiges cylindriques peut quant à lui être solidaire d'une courroie guidée sur des poulies montées dans le logement de la cassette et dont l'une est entraînée en rotation par des moyens moteurs.

Selon encore une autre caractéristique de l'invention, la plaque d'obturation de l'extrémité ouverte de la cassette est déplacée entre ses deux positions de fonctionnement par un organe mobile qui est monté à l'entrée de la zone de stockage tampon et qui est actionné par le chariot des premiers moyens de transfert, lorsque ce chariot arrive à l'entrée de la zone tampon.

Dans sa position de sortie des cuvettes, la plaque d'obturation de l'extrémité ouverte de la cassette coopère avec une butée prévue dans le logement de la cassette pour retenir cette cassette en place dans le logement.

Selon encore une autre caractéristique de l'invention, la zone de stockage tampon comprend des moyens de support de cuvettes s'étendant dans le prolongement des nervures de support de cuvettes d'une cassette placée dans le logement précité, pour recevoir des cuvettes provenant des rangées de cuvettes contenues dans la cassette, et les seconds moyens de transfert des cuvettes comprennent au moins un peigne latéral dont les dents s'étendent transversalement entre les moyens de support de cuvettes de la zone de stockage tampon pour pousser simultanément toutes les cuvettes de cette zone vers un convoyeur à courroie qui reçoit les cuvettes d'extrémité des rangées de la zone de stockage tampon et les amène une à une vers des moyens d'entrée des cuvettes dans l'appareil d'analyse.

Dans un mode de réalisation préféré de l'invention, les rangées de cuvettes sont verticalement superposées dans la cassette et dans la zone de stockage tampon et le convoyeur à courroie qui se trouve à la sortie de la zone de stockage tampon est vertical et forme un moyen de dépilage.

Le ou les peignes des seconds moyens de transfert des cuvettes sont portés par un coulisseau qui est déplaçable sur le rail de guidage du chariot des premiers moyens de transfert, ce coulisseau étant poussé pas à pas vers la sortie de la zone de stockage tampon par le chariot des premiers moyens de transfert pour vider cette zone de stockage tampon et étant ramené par ce chariot à l'entrée de la zone de stockage tampon quand celle-ci est vide.

Dans un mode de réalisation préféré de l'invention, le coulisseau et le chariot précités sont solidarisés l'un à l'autre par attraction magnétique quand le chariot est amené à l'entrée de la zone de stockage tampon, une butée étant prévue à l'entrée de cette zone pour retenir le coulisseau et le désolidariser du chariot quand celui-ci est ramené à l'entrée du logement de réception de cassette.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- La figure 1 est une vue schématique de face d'un dispositif d'alimentation d'un appareil d'analyse en cuvettes de réaction, une cassette pleine étant en place dans ce dispositif ;
- La figure 2 représente le même dispositif d'alimentation, sans la cassette ;
- La figure 3 est une vue schématique en perspective d'une demi-cassette ;
- La figure 4 est une vue schématique en perspective et à plus grande échelle d'une cuvette de réaction;
- La figure 5 est une vue de face d'un élément d'obturation d'une cassette selon l'invention ;
- La figure 6 est une vue schématique en perspective d'un poussoir de la cassette;
- La figure 7 est une vue schématique partielle en perspective et en coupe représentant l'agencement des cuvettes dans une cassette selon l'invention;
- La figure 8 est une vue schématique partielle en perspective représentant un poussoir en place dans la cassette ;
- La figure 9 est une vue schématique en perspective et à plus grande échelle représentant les moyens de retenue des billes dans les cuvettes dans une cassette selon l'invention;
- La figure 10 est une vue schématique de face et à plus grande échelle de la zone de stockage tampon et des moyens de dépilage;
- La figure 11 est une vue schématique partielle à plus grande échelle de la sortie des moyens de dépilage.

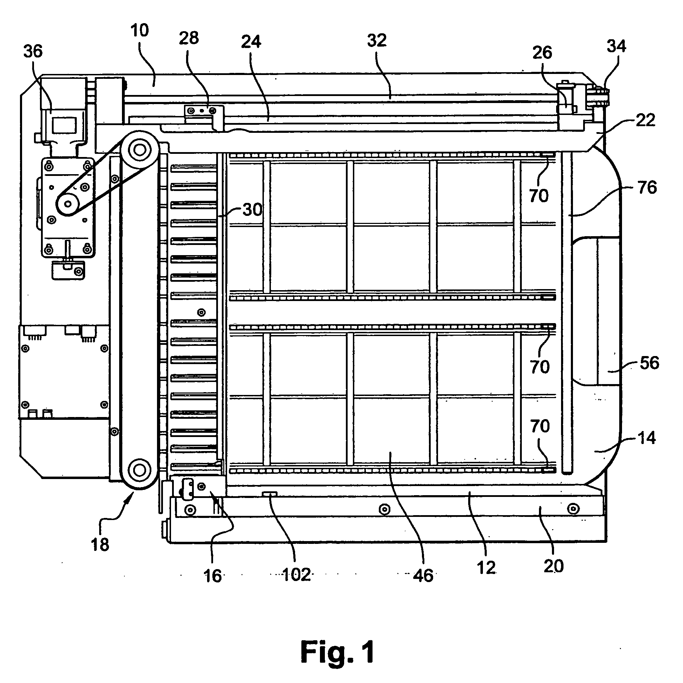

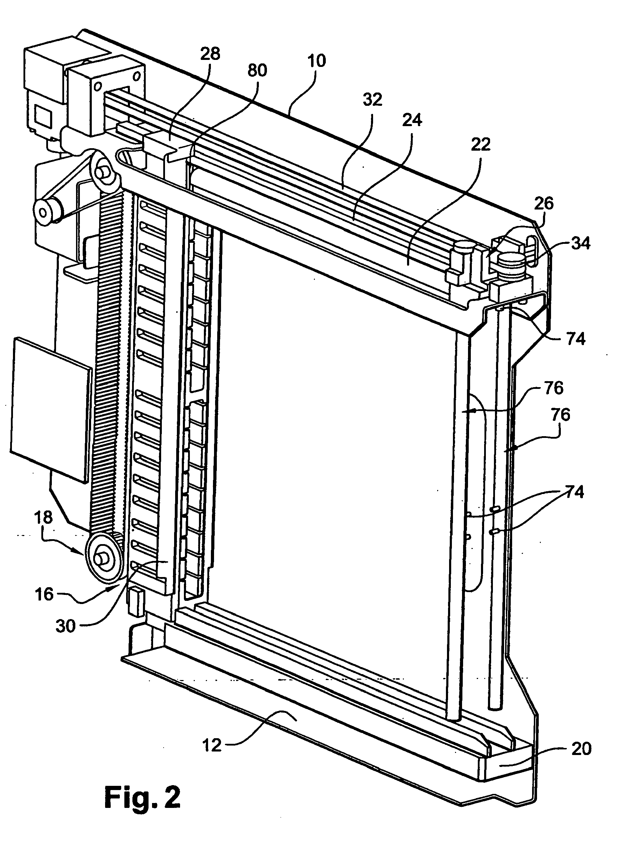

Le dispositif d'alimentation en cuvettes de réaction représenté aux figures 1 et 2 fait partie d'un appareil automatique d'analyse, en particulier pour la détermination des temps de modification de l'état physique d'un milieu, et comprend essentiellement une platine 10 destinée à être fixée sur l'appareil et portant des moyens 12 formant le logement d'une cassette 14 contenant un agencement ordonné de cuvettes de réaction, des moyens 16 formant une zone de stockage tampon de cuvettes de réaction, et des moyens 18 d'amenée des cuvettes de réaction une par une jusqu'à un point d'entrée dans l'appareil d'analyse.

Les moyens 12 formant le logement de réception d'une cassette 14 comprennent deux rails horizontaux 20, 22 superposés verticalement dans lesquels sont guidés les bords horizontaux inférieur et supérieur de la cassette 14, des moyens de détrompage étant prévus sur l'un des rails de guidage et sur le bord correspondant de la cassette pour interdire un montage de la cassette 14 à l'envers dans son logement.

Un détecteur 102 de présence de cassette dans le logement peut également être prévu dans la partie avant du rail inférieur 20, pour constater qu'une cassette 14 a été montée dans le bon sens dans le logement et a été poussée jusqu'à l'entrée de la zone 16 de stockage tampon.

La partie supérieure du logement comporte également un rail horizontal 24, s'étendant au-dessus du rail supérieur de guidage 22 sur toute la longueur du logement de la cassette et sur toute la longueur également de la zone 16 de stockage tampon et des moyens 18 d'amenée des cuvettes à l'entrée de l'appareil.

Ce rail 24 sert au support et au guidage d'un chariot 26 portant des moyens de déplacement des cuvettes de réaction dans la cassette 14 jusqu'à la zone 16 de stockage tampon, et d'un coulisseau 28 portant des peignes verticaux 30 de déplacement des cuvettes de réaction dans la zone 16 de stockage tampon.

Le chariot 26 est fixé à une courroie horizontale 32 qui passe sur deux poulies opposées 34, dont une seule située à l'extrémité arrière du logement de la cassette est visible aux figures 1 et 2, l'autre poulie étant entraînée en rotation par des moyens moteurs 36 pour déplacer en va et vient, au moyen de la courroie 32, le chariot 26 entre l'extrémité arrière du logement de la cassette et l'extrémité avant de la zone 16 de stockage tampon, comme cela sera décrit plus en détail dans ce qui suit.

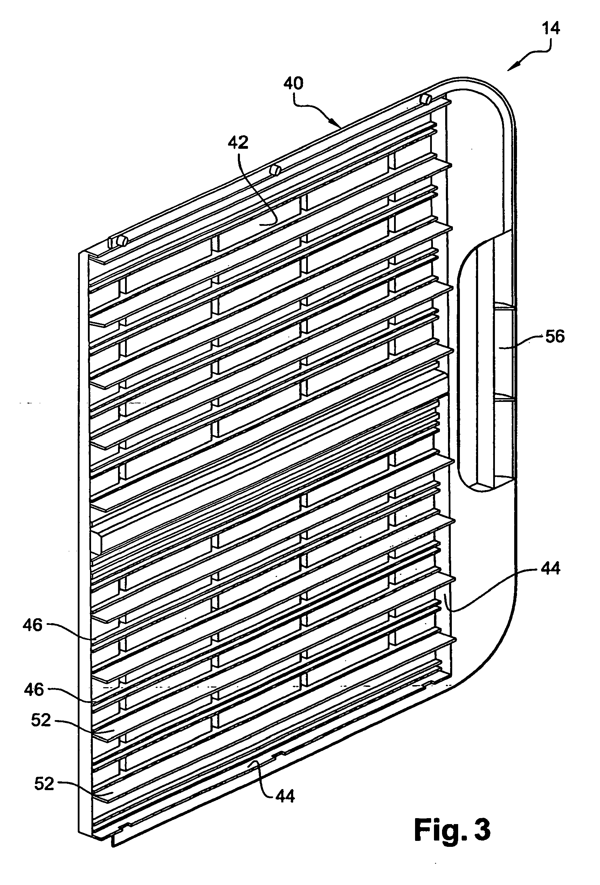

La cassette 14 contenant un agencement ordonné de cuvettes de réaction va maintenant être décrite en référence aux figures 3 à 9.

Cette cassette est de forme parallélépipédique rectangle et est formée par juxtaposition et fixation de deux demi coquilles 40, dont l'une est représentée en détail en figure 3, ces deux demi-coquilles étant réalisées par exemple en un matériau plastique tel que du polystyrène moulé par injection.

Chaque demi-coquille 40 comprend essentiellement une paroi plane 42 formant une des grandes faces latérales de la cassette et un rebord périphérique 44 qui coopère avec le rebord 44 de l'autre demi-coquille pour former les petites faces horizontales et verticales de la cassette lorsque les deux demi-coquilles sont assemblées.

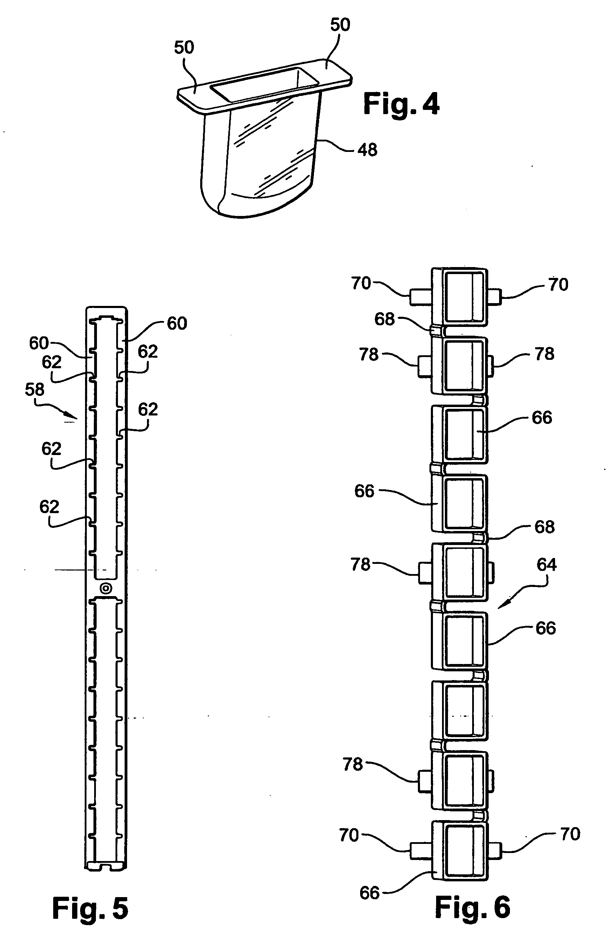

Des nervures horizontales longitudinales 46 sont formées en saillie sur les faces internes des demi-coquilles 40 pour supporter les rangées de cuvettes de réaction 48 telles que celle représentée en figure 4, chaque cuvette comprenant deux rebords latéraux opposés 50 à son extrémité supérieure ouverte, par lesquels elle repose sur une nervure 46 d'une demi-coquille 40 et sur une nervure 46 située au même niveau de l'autre demi-coquille 40.

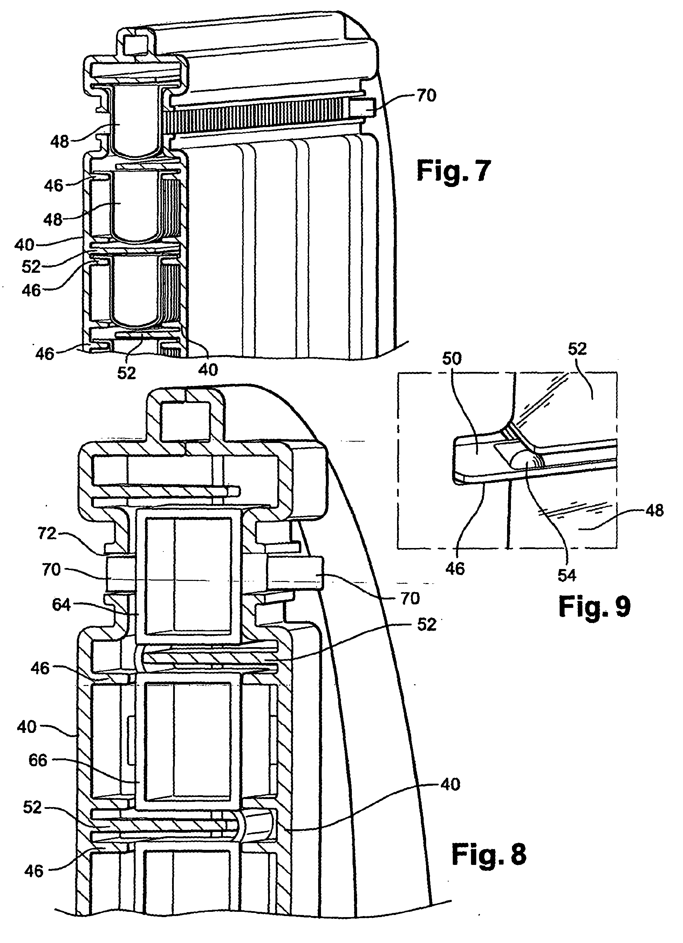

La disposition de rangées de cuvettes 48 sur les nervures 46 des demi-coquilles a été illustrée schématiquement en figure 7.

Des ailettes longitudinales horizontales 52 sont également formées en saillie sur les faces internes des demi-coquilles 42, en alternance avec les nervures 46 de façon à ce que chaque ailette 52 s'étende un peu au-dessus d'une cuvette 48 et obture l'extrémité supérieure ouverte de cette cuvette d'une façon incomplète mais suffisante pour empêcher une bille ferromagnétique 54 contenue dans la cuvette de sortir de celle-ci, comme illustré schématiquement en figure 9.

On peut par exemple, comme représenté aux figures 7 et 8, former ces ailettes 52 en alternance sur une demi-coquille 40 pour une rangée de cuvettes 48 et sur l'autre demi-coquille 40 pour la rangée supérieure ou inférieure de cuvettes 48.

L'extrémité verticale arrière de la cassette 14 est fermée par les rebords 44 des demi-coquilles 40 et comporte une poignée 56 permettant de manipuler commodément la cassette. L'extrémité verticale avant de la cassette est ouverte, pour permettre une sortie des rangées de cuvettes 48 vers la zone 16 de stockage tampon lorsque la cassette est en place dans le logement du dispositif d'alimentation, et est équipée d'un obturateur 58 (figure 5) qui est formé d'un cadre allongé dont les dimensions correspondent à celles de l'extrémité verticale avant de la cassette, les côtés verticaux 60 de ce cadre comportant des encoches 62 sur leurs bords internes correspondant aux rebords 50 des cuvettes de réaction 48 contenues dans la cassette.

L'obturateur 58 est guidé en translation verticale entre les deux demi-coquilles 40 et est monté, lorsque la cassette est remplie de cuvettes de réaction, dans une position de fermeture interdisant la sortie des cuvettes. Cela permet de manipuler la cassette en évitant la chute des cuvettes, quelle que soit l'orientation de la cassette.

Pour permettre la sortie des cuvettes lorsque la cassette a été mise en place dans le logement du dispositif d'alimentation, l'obturateur 58 peut être déplacé par un élément qui sera décrit plus en détail dans ce qui suit pour que les encoches 62 de ses bords 60 soient alignées avec les rebords 50 des cuvettes 48 reposant sur les nervures 46 des demi-coquilles 40.

Un poussoir 64 représenté en figure 6 est monté à l'intérieur de la cassette, à son extrémité arrière, pour pousser les rangées de cuvettes contenues dans la cassette vers l'extrémité avant de celle-ci.

Ce poussoir 64 s'étend sur toute la hauteur de la cassette ou, dans l'exemple représenté, sur la moitié de cette hauteur, deux poussoirs 64 étant alors placés dans l'alignement l'un de l'autre à l'extrémité arrière de la cassette.

Chaque poussoir 64 comprend une série de plots rectangulaires ou carrés 66 dont le nombre correspond au nombre de rangées de cuvettes que le poussoir 64 doit déplacer à l'intérieur de la cassette et dont la forme correspond au contour d'une cuvette de réaction. Ces différents plots 66 sont reliés les uns aux autres par des pontets 68 disposés en alternance sur un côté vertical du poussoir 64 et sur son autre côté vertical, pour permettre un montage du poussoir sur les ailettes 52 qui sont formées en alternance sur les deux demi-coquilles 40, comme représenté schématiquement en figure 8.

Cette configuration permet un support et un guidage correct de chaque poussoir 64 à l'intérieur de la cassette 14.

Les plots d'extrémité 66 du poussoir 64 portent des oreilles latérales 70 qui s'étendent au travers de fentes 72 des parois latérales des demi-coquilles 40 et qui sont destinées à venir en prise avec des doigts 74 de deux tiges verticales 76 portées par le chariot 26, ces deux tiges 76 s'étendant de part et d'autre de la cassette 14 de façon à ce que leurs doigts 74 puissent venir en butée sur les oreilles 70 des poussoirs 64 et déplacer ces derniers en direction de la zone 16 de stockage tampon.

Les tiges 76 sont pivotantes sur un quart de tour autour de leur axe sur le chariot 26, entre deux positions où les doigts 74 sont respectivement perpendiculaires aux parois 42 de la cassette et orientés les uns vers les autres, et où ils sont parallèles aux parois 42, respectivement.

Des cames portées par les extrémités supérieures des tiges 76 viennent en appui sur des butées prévues aux extrémités de la course du chariot 26 pour faire pivoter les tiges 76 d'une de leurs positions dans l'autre et inversement.

Comme représenté en figure 6, d'autres plots 66 du poussoir 64 peuvent porter des oreilles latérales 78, de plus faible dimension que les oreilles 70 des plots d'extrémité 66.

Pour l'assemblage de la cassette, les poussoirs 64 sont mis en place sur une demi-coquille 40, à l'extrémité arrière de cette demi-coquille, puis la seconde demi-coquille est montée sur la première, le montage ayant lieu par exemple par clipsage de façon à ce que la cassette ne soit plus démontable sans rupture de certaines de ses parties. La cassette ainsi assemblée peut être chargée de cuvettes de réaction, jusqu'à être complètement remplie. Dans l'exemple de réalisation représenté, la cassette 14 comprend dix huit rangées superposées de cinquante six cuvettes, soit un total de 1008 cuvettes contenant chacune une bille 54.

Lorsque la cassette est remplie de cuvettes, on met l'obturateur 58 en place à l'extrémité avant de la cassette dans une position où il interdit la sortie des cuvettes. On voit en figure 5 que les extrémités verticales de l'obturateur 58 ne sont pas identiques, de sorte que l'obturateur peut jouer le rôle d'un détrompeur qui permet l'introduction complète de la cassette dans le logement du dispositif d'alimentation des figures 1 et 2 quand la cassette est positionnée correctement et qui interdit cette introduction complète lorsque la cassette est à l'envers.

L'entrée de la zone 16 de stockage tampon comprend un élément vertical 80 qui est guidé en translation verticale sur cette extrémité de la zone 16 et qui coopère avec l'obturateur 58 de l'extrémité avant de la cassette pour déplacer cet obturateur entre ses positions d'ouverture et de fermeture de la sortie des cuvettes, cet élément mobile 80 étant lui-même déplacé en translation par le chariot 26 au moyen d'un système de rampes qui coopèrent avec l'élément mobile 80 pour le déplacer verticalement dans un sens et dans l'autre. Ainsi, lorsque le chariot 26 arrive à l'entrée, de la zone 16 de stockage tampon, il abaisse l'élément 80, ce qui place l'obturateur 58 dans une position interdisant la sortie des cuvettes de la cassette. Lorsque le chariot 26 est ramené en position arrière représentée aux figures 1 et 2, pour le déchargement d'une cassette pleine qui vient d'être placée dans le logement du dispositif d'alimentation, l'élément mobile 80 est remonté par le système de rampes du chariot, ce qui place l'obturateur 58 de la nouvelle cassette dans une position autorisant la sortie des cuvettes. L'obturateur 58 se trouve alors dans une position haute où son extrémité supérieure peut coopérer avec une butée portée par l'extrémité avant de la zone 16 de stockage tampon, ce qui verrouille la cassette 14 dans son logement du dispositif d'alimentation et interdit de la retirer de ce logement.

La zone 16 de stockage tampon comprend elle-même une série de nervures longitudinales horizontales 84 de support des rebords 50 des cuvettes de réaction 48, dans le prolongement des nervures 46 de la cassette 14. La zone de stockage tampon 16 peut ainsi comprendre dix huit rangées verticalement superposées de cuvettes de réaction, chaque rangée comprenant par exemple neuf cuvettes de réaction.

Les peignes 30 portés par le coulisseau 28 s'étendent transversalement derrière la dernière cuvette de réaction 48 de chaque rangée et permettent de déplacer les cuvettes de réaction 48 vers l'avant dans la zone 16 de stockage tampon quand le coulisseau 28 est déplacé vers l'avant par le chariot 26 précité.

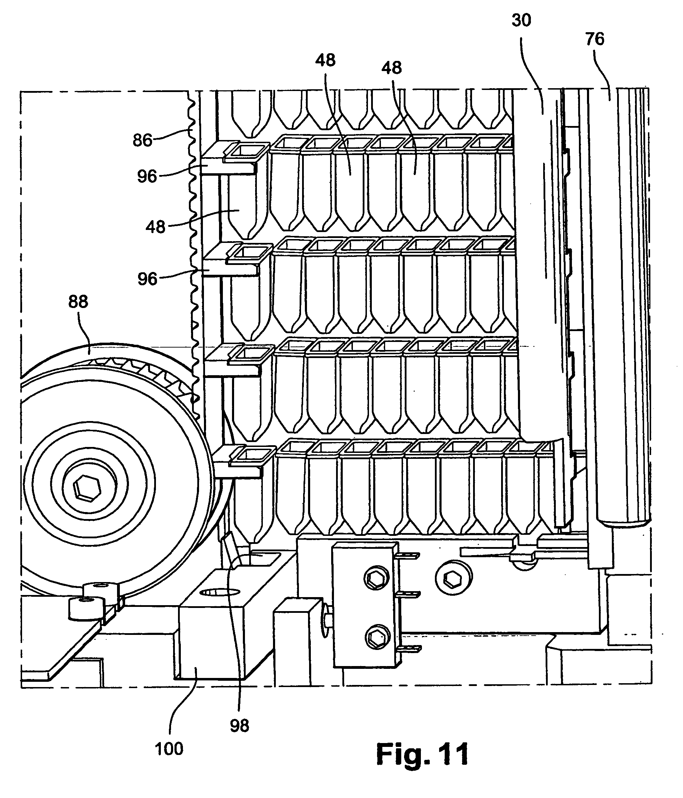

A l'extrémité avant de la zone 16 de stockage tampon se trouvent les moyens 18, qui comprennent un dépileur à convoyeur vertical formé d'une courroie crantée 86 passant sur deux poulies 88 verticalement alignées, la poulie supérieure 88 étant entraînée en rotation par un moteur électrique 90 et par une courroie crantée 92 passant sur une poulie solidaire de la poulie supérieure 88 et sur une poulie 94 solidaire de l'arbre de sortie du moteur 90. Le brin vertical de la courroie 86, qui s'étend le long de la sortie de la zone 16 de stockage tampon, porte des supports en U 96 orientés vers la zone 16 et destinés chacun à recevoir une cuvette de réaction 48 comme représenté en figure 11.

La descente pas à pas de ce brin de la courroie 86 permet de déposer les cuvettes de réaction une à une dans un logement 98 d'un bloc de réception 100 qui est ensuite déplacé par des moyens appropriés jusqu'à un point d'entrée des cuvettes de réaction dans l'appareil d'analyse automatique.

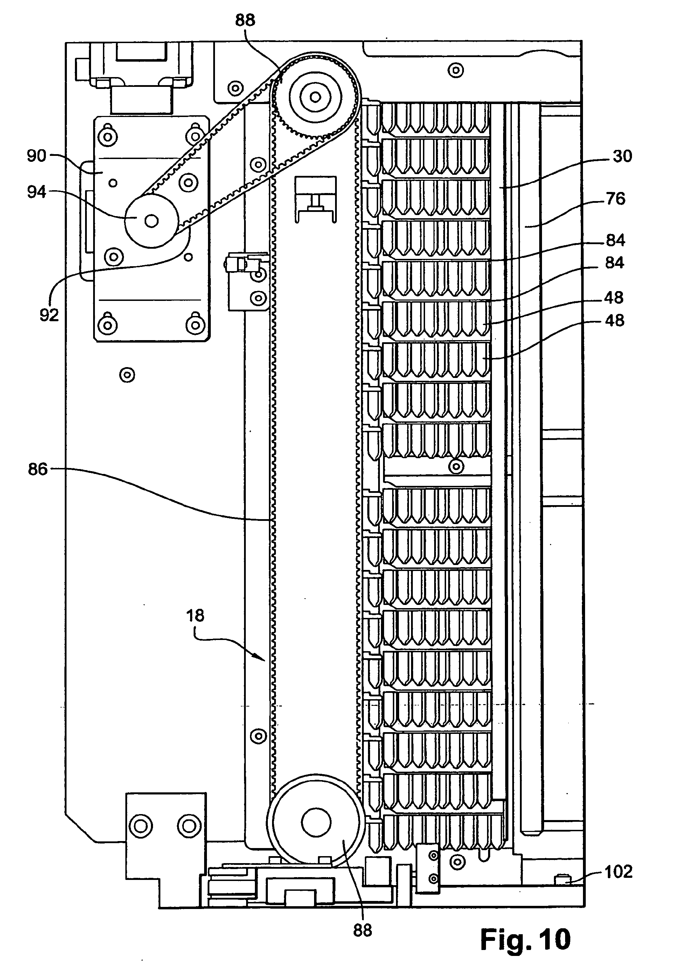

Le dispositif d'alimentation en cuvettes de réaction qui vient d'être décrit fonctionne de la façon suivante:
Lorsqu'une cassette 14 correctement placée dans les rails 20, 22 de son logement dans le dispositif d'alimentation a été vidée de ses cuvettes de réaction, le chariot 26 portant les tiges cylindriques 76 se trouve à l'avant de ce logement et juste à l'entrée de la zone 16 de stockage tampon comme représenté dans les figures 10 et 11. Dans cette position, la zone 16 de stockage tampon est entièrement remplie de cuvettes de réaction 48.
Lorsque toutes les cuvettes de réaction portées par les supports 96 de la courroie 86 ont été déposées l'une après l'autre dans le bloc de réception 100 et amenées à l'entrée de l'appareil d'analyse automatique, le moteur 90 est commandé pour remonter la courroie 86 et les supports 96 dans une position initiale représentée en figure 10 où chaque support 96 est dans le prolongement d'une rangée de cuvettes de réaction 48 de la zone 16 de stockage tampon. Le chariot 26 est alors déplacé d'un pas vers l'avant et pousse d'un pas le coulisseau 28 vers la sortie de la zone 16 de stockage tampon, ce qui a pour effet de déposer une cuvette de réaction 48 sur chaque support 96 de la courroie 86. Le moteur 90 est alors commandé à nouveau pour faire descendre les supports 96 sur un pas et déposer une cuvette de réaction 48 dans le logement 98 du bloc de réception 100.

Le processus peut se poursuivre jusqu'à ce que toutes les cuvettes de réaction contenues dans la zone 16 de stockage tampon aient été déposées une par une dans le bloc de réception 100, ce qui assure un fonctionnement continu de l'appareil d'analyse sur environ 30 minutes.

Pendant ce temps, la cassette vide peut être retirée de son logement dans le dispositif d'alimentation et remplacée par une cassette 14 remplie de cuvettes de réaction. Il suffit pour cela de tirer sur la poignée 56 de la cassette vide et de la sortir des rails 20, 22 puis de prendre une cassette 14 remplie de cuvettes de réaction par sa poignée 56 et de l'introduire dans les rails 20, 22 du logement du dispositif d'alimentation.

Les moyens de détrompage interdisent de placer la cassette 14 à l'envers. Lorsque la cassette 14 a été introduite dans son logement jusqu'à l'entrée de la zone 16 de stockage tampon, un détecteur 102, visible aux figures 1 et 10 confirme le montage correct de la cassette dans le logement du dispositif d'alimentation et autorise la commande du déplacement du chariot 26 par les moyens moteurs 36.

Le chariot 26, qui avait été avancé jusqu'à la sortie de la zone 16 de stockage tampon pour la vider de ses cuvettes, est ramené vers l'arrière par la courroie 32 jusqu'à l'extrémité arrière de la cassette 14 dans la position représentée aux figures 1 et 2. Pendant ce mouvement de retour, il commande le déplacement de l'obturateur 58 de la cassette pour le placer dans une position autorisant la sortie des cuvettes de réaction et verrouillant la cassette dans son logement du dispositif d'alimentation.

Par ailleurs, pendant ce mouvement de retour, les tiges cylindriques 76 portées par le chariot 26 sont dans une position angulaire autour de leur axe où les doigts 74 s'étendent parallèlement aux rails 20 et 22, ce qui permet de déplacer les tiges 76 le long des parois latérales 42 de la cassette 14 sans interférer avec les oreilles 70 des poussoirs 64, qui se trouvent en position arrière dans la cassette comme représenté en figure 1.

Quand le chariot 26 arrive en fin de course en position arrière, des cames portées par les extrémités supérieures des tiges 76 viennent en contact avec des butées correspondantes portées par le rail 22 et font pivoter d'un quart de tour les tiges 76 de façon à ce que les doigts 74 s'étendent l'un vers l'autre et perpendiculairement aux rails 20 et 22, comme représenté en figure 2. Dans cette position, les doigts 74 vont venir s'appliquer sur les oreilles 70 des poussoirs 64 quand le chariot 26 sera déplacé vers l'avant.

L'arrivée du chariot 26 dans sa position arrière, après ouverture de la sortie de la cassette par l'obturateur 58 et verrouillage de la cassette dans son logement du dispositif d'alimentation, autorise le retour du chariot 26 vers sa position avant. Ce déplacement du chariot 26 provoque un déplacement vers l'avant des rangées de cuvettes contenues dans la cassette 14 qui sont entraînées par les poussoirs 64, jusqu'à ce que la zone 16 de stockage tampon soit à nouveau remplie de cuvettes de réaction. Ensuite, lorsque toutes les cuvettes de réaction portées par les supports 96 de la courroie 86 des moyens de dépilage 18 ont été déposées l'une après l'autre dans le bloc de réception 100, et lorsque les supports 96 ont été ramenés dans l'alignement des rangées de cuvettes de réaction de la zone 16, une nouvelle avance d'un pas du chariot 26 permet de regarnir les supports 96, et ainsi de suite.

Lorsque le chariot 26 arrive à l'entrée de la zone 16 de stockage tampon, les cames portées par des extrémités supérieures des tiges cylindriques 76 viennent s'appuyer sur des butées fixes qui font tourner les tiges 76 sur un quart de tour autour de leur axe pour orienter les doigts 74 parallèlement aux rails 20 et 22. La butée du chariot 26 sur le coulisseau 28 permet de déplacer ce dernier vers l'avant pas à pas, pour vider la zone 16 de stockage tampon. Lorsque cette zone est vide, le retour du chariot 26 vers sa position arrière ramène également le coulisseau 28 à l'entrée de la zone 16 de stockage tampon. Pour cela, des aimants permanents peuvent être montés sur le chariot 26 et/ou sur le coulisseau 28 pour les solidariser par attraction magnétique lorsqu'ils sont au contact l'un de l'autre. Lorsque le coulisseau 28 a été ramené par le chariot 26 à l'entrée de la zone 16 de stockage tampon, il est retenu par une butée fixe qui l'empêche de se déplacer davantage vers l'arrière lorsque le chariot 26 est ramené vers la position de la figure 1.

Par ailleurs, l'arrivée du chariot 26 dans sa position avant à l'entrée de la zone 16 de stockage tampon a également pour effet de commander le déplacement de l'obturateur 58 dans sa position de fermeture de la sortie de la cassette 14 et de déverrouillage de cette cassette, qui peut alors être retirée de son logement du dispositif d'alimentation.

## Revendications

1. Cassette d'alimentation en cuvettes de réaction pour un appareil d'analyse automatique, en particulier de détermination des temps de modification de l'état physique d'un milieu, **caractérisée en ce qu'**elle comprend :
- deux parois latérales parallèles (42) assemblées fixement l'une à l'autre et comportant des moyens (46) de support de rangées de cuvettes de réaction (48),
- une face d'extrémité fermée, formée par des rebords (44) des parois latérales (42) et empêchant les cuvettes de sortir de la cassette,
- et une face d'extrémité ouverte, opposée à la face d'extrémité fermée et équipée d'un obturateur coulissant (58), déplaçable entre une position de sortie des cuvettes de réaction et une position de retenue des cuvettes.

2. Cassette selon la revendication 1, **caractérisée en ce que** les moyens de support des rangées de cuvettes (48) comprennent des nervures (46) formées en saillie sur les faces internes des parois latérales (42) et sur lesquelles reposent des rebords latéraux (50) des cuvettes (48) et **en ce que** les faces internes des parois latérales (42) comportent des ailettes (52) parallèles aux nervures (46) de support des cuvettes et s'étendant au-dessus de celles-ci pour fermer au moins partiellement les extrémités supérieures ouvertes des cuvettes (48) portées par ces nervures et empêcher la sortie de billes (54) contenues dans les cuvettes.

3. Cassette selon l'une des revendications précédentes, **caractérisée en ce qu'**elle comprend au moins un poussoir formé par une plaquette transversale (64) s'étendant entre les parois latérales (42) et guidée en translation sur des moyens (46, 52) de support ou de guidage des cuvettes, cette plaquette comportant des oreilles ou pattes latérales (70) s'étendant à travers des fentes des parois latérales (42) de la cassette et formant des moyens d'entraînement de la plaquette d'une extrémité à l'autre de la cassette pour déplacer simultanément toutes les rangées de cuvettes (48) vers l'extrémité ouverte de la cassette.

4. Cassette selon l'une des revendications précédentes, **caractérisée en ce que** l'extrémité fermée de la cassette comprend une poignée (56) de manipulation.

5. Cassette selon l'une des revendications précédentes, **caractérisée en ce qu'**elle est réalisée en métal ou en matériau plastique moulé par injection ou thermoformé.

6. Appareil automatique d'analyse, en particulier pour la détermination des temps de modification de l'état physique d'un milieu, comprenant des moyens d'alimentation en cuvettes de réaction (48), **caractérisé en ce que** ces moyens d'alimentation comprennent :
- des moyens (10, 20, 22) formant un logement de réception d'une cassette (14) selon l'une des revendications précédentes,
- des moyens prévus à une extrémité de ce logement et formant une zone (16) de stockage tampon de cuvettes de réaction,
- des premiers moyens (26, 32, 36, 76) de transfert pour déplacer les cuvettes (48) de la cassette (14) placée dans le logement jusque dans la zone (16) de stockage tampon,
- et des seconds moyens (18, 28, 30) pour déplacer les cuvettes de la zone (16) de stockage tampon jusqu'à un point d'entrée des cuvettes dans l'appareil d'analyse.

7. Appareil selon la revendication 6, **caractérisé en ce que** les moyens d'entraînement du ou des poussoirs (64) dans la cassette (14) comprennent un chariot (26) déplacé sur un rail de guidage longitudinal (24) par des moyens moteurs (32, 36), ce chariot portant au moins une tige cylindrique transversale (76) s'étendant le long des rangées de cuvettes, perpendiculairement à ces rangées, et portant des doigts (74) destinés à coopérer avec les oreilles latérales (70) du ou des poussoirs (64) pour les déplacer d'un bout à l'autre des rangées de cuvettes et faire sortir des cuvettes (48) de la cassette (14).

8. Appareil selon la revendication 7, **caractérisé en ce que** la ou chaque tige cylindrique (76) est montée pivotante autour de son axe sur le chariot (26), entre une position de travail ou les doigts (74) sont en prise avec les oreilles latérales (70) du ou des poussoirs (64) et une position de repos où les doigts (74) sont écartés des oreilles latérales (70) du ou des poussoirs (64).

9. Appareil selon la revendication 7 ou 8, **caractérisé en ce que** la ou chaque tige cylindrique (76) porte des cames coopérant avec des butées fixes du logement de la cassette (14), pour faire pivoter la tige (76) d'une de ses positions dans l'autre et inversement.

10. Appareil selon l'une des revendications 7 à 9, **caractérisé en ce que** les moyens d'entraînement du chariot (26) comprennent une courroie (32) guidée sur des poulies (34) dont l'une est entraînée en rotation par des moyens moteurs (36).

11. Appareil selon l'une des revendications 6 à 10, **caractérisé en ce que** la plaque (58) d'obturation de la sortie de la cassette (14) est déplacée entre ses deux positions par un organe mobile (80) qui est monté à l'entrée de la zone (16) de stockage tampon et qui est actionné par le chariot (26) des premiers moyens de transfert, ladite plaque d'obturation (58) coopérant dans sa position de sortie des cuvettes (48) avec une butée prévue dans le logement de la cassette (14), pour verrouiller la cassette en place dans son logement.

12. Appareil selon l'une des revendications 6 à 11, **caractérisé en ce que** la zone (16) de stockage tampon comprend des moyens de support de cuvettes s'étendant dans le prolongement des moyens (46) de support de cuvettes d'une cassette (14) placée dans le logement précité, pour recevoir des cuvettes provenant des rangées de cuvettes contenues dans la cassette, et **en ce que** les seconds moyens de transfert des cuvettes comprennent au moins un peigne latéral (30) dont les dents s'étendent transversalement entre les moyens de support de cuvettes de la zone (16) de stockage tampon pour pousser simultanément toutes les cuvettes de cette zone vers un convoyeur à courroie (86) qui reçoit les cuvettes d'extrémité des rangées de cuvettes de la zone (16) de stockage tampon et les amène une à une vers un point d'entrée des cuvettes dans l'appareil d'analyse.

13. Appareil selon l'une des revendications 6 à 12, **caractérisé en ce que**, dans la cassette (14) et dans la zone (16) de stockage tampon, les rangées de cuvettes (48) sont verticalement superposées et **en ce que** le convoyeur à courroie à la sortie de la zone (16) de stockage tampon est vertical, la courroie (86) du convoyeur portant une série de supports (96) de cuvettes, le nombre de ces supports étant égal au nombre de rangées de cuvettes dans la zone (16) de stockage tampon.

14. Appareil selon la revendication 12 ou 13, **caractérisé en ce que** le ou les peignes (30) des seconds moyens de transfert sont portés par un coulisseau (28) déplaçable sur le rail (24) de guidage du chariot (26) des premiers moyens de transfert, ce coulisseau (28) étant poussé pas à pas vers la sortie de la zone (16) de stockage tampon par le chariot (26) des premiers moyens de transfert pour vider la zone (16) de stockage tampon et étant ramené par ce chariot (26) à l'entrée de la zone de stockage tampon quand celle-ci est vide.

15. Appareil selon la revendication 14, **caractérisé en ce que** le coulisseau (28) et le chariot (26) sont solidarisés par attraction magnétique quand le chariot (26) est amené à l'entrée de la zone (16) de stockage tampon, une butée étant prévue à l'entrée de cette zone pour retenir le coulisseau (28) et le désolidariser du chariot (26) quand ce chariot est ramené à l'entrée du logement de réception de cassette.

## Claims

1. Cassette for supplying reaction cuvettes to an automatic analysis machine, **characterised in that** it comprises:
- two parallel side walls (42) fixedly assembled with each other and comprising means (46) for supporting rows of reaction cuvettes (48),
- one closed end face, formed by edges (44) of the side walls (42) and preventing the cuvettes from coming out of the cassette,
- and one open end face, opposite the closed end face and fitted with a sliding seal (58), movable between a cuvette exit position and a cuvette holding position.

2. Cassette according to claim 1, **characterised in that** the means for supporting the rows of cuvettes (48) comprise ribs (46) formed protruding on the inner faces of the side walls (42) of the cassette and whereon the side edges (50) of the cuvettes (48) are supported and the inner faces of the side walls (42) of the cassette comprise fins (52) parallel with the ribs (46) for supporting the cuvettes and extending over same to close the open upper ends of the cuvettes (48) supported by said ribs at least partially and prevent the beads (54) contained in the cuvettes from coming out.

3. Cassette according to any of the above claims, **characterised in that** it comprises at least one pushing device formed by a transverse insert (64) extending between the side walls (42) and which is guided in translation on the means (46, 52) for supporting the cuvettes, said insert comprising side lugs or tabs (70) extending through the slots of the side walls (42) of the cassette to form means for actuating the insert from one end to the other of the cassette so as to move all the rows of cuvettes (48) to the open end of the cassette simultaneously.

4. Cassette according to any of the above claims, **characterised in that** the closed end of the cassette comprises a handle (56) for handling.

5. Cassette according to any of the above claims, **characterised in that** it is made of metal or injection-moulded or thermoformed plastic.

6. Automatic analysis machine, particularly for determining the time required for the physical state of a medium to change, comprising means for supplying reaction cuvettes (48), **characterised in that** said supply means comprise:
- means (10, 20, 22) forming a housing to receive a cassette (14) according to any of the above claims,
- means provided at one end of said housing and forming a cuvette buffer storage zone (16),
- first transfer means (26, 32, 36, 76) for moving the cuvettes (48) from the cassette (14) placed in the housing to the buffer storage zone (16),
- and second transfer means (18, 28, 30) for moving the cuvettes from the buffer storage zone (16) to a point at which the cuvettes enter the analysis machine.

7. Machine according to claim 6, **characterised in that** the means for actuating the pushing device(s)(64) in the cassette (14) comprise a carriage (26), moved on a longitudinal guide rail (24) by motor means (32, 36), said carriage supporting at least one transverse cylindrical rod (76) extending along the rows of cuvettes, perpendicular to these rows and supporting tappets (74) to engage with the side lugs (70) of the pushing device(s) (64) to move same from one end of the cuvette rows to the other and release the cuvettes (48) from the cassette (14).

8. Machine according to claim 7, **characterised in that** the or each cylindrical rod (76) is pivotably mounted about the axis thereof on the carriage (26), between a working position where the tappets (74) are engaged with the side lugs (70) of the pushing device(s) (64) and an idle position where the tappets (74) are separated from the side lugs (70) of said pushing device(s) (64).

9. Machine according to claim 7 or 8, **characterised in that** the or each cylindrical rod (76) support cams engaging with fixed abutments of the housing of the cassette (14), to pivot the rod (76) from one of the positions thereof to the other and conversely.

10. Machine according to any of claims 7 to 9, **characterised in that** the means for actuating the carriage (26) comprise a belt (32) guided on pulleys (34) one of which is rotated by motor means (36).

11. Machine according to any of claims 6 to 10, **characterised in that** the sealing plate (58) of the exit of the cassette (14) is moved between the two operating positions thereof by a movable member (80) which is mounted at the entry of the buffer storage zone (16) and is actuated by the carriage (26) of the first transfer means, said sealing plate (58) engaging in the cuvette (48) exit position thereof with an abutment provided in the cassette (14) housing to hold said cassette in position in the housing.

12. Machine according to any of claims 6 to 11, **characterised in that** the buffer storage zone (16) comprises means for supporting the cuvettes extending from the means (46) for supporting cuvettes of a cassette (14) placed in said housing, to receive cuvettes from the rows of cuvettes contained in the cassette, and **in that** the second cuvette transfer means comprise at least one side comb (30) wherein the teeth extend transversely between the means for supporting cuvettes in the buffer storage zone (16) to push all the cuvettes from this zone simultaneously to a conveyor belt (86) receiving the end cuvettes of the rows of cuvettes of the buffer storage zone (16) and conveying same one by one to means for entering the cuvettes in the analysis machine.

13. Machine according to any of claims 6 to 12, **characterised in that**, in the cassette (14) and in the buffer storage zone (16), the rows of cuvettes (48) are stacked vertically and **in that** the conveyor belt situated at the exit of the buffer zone (16) is vertical, the belt (86) of the conveyor supporting a series of cuvette supporting members (96), the number of these supporting members being equal to the number of rows of cuvettes in the buffer storage zone (16).

14. Machine according to claim 12 or 13, **characterised in that** the comb(s) (30) of the second transfer means is/are supported by a slide (28) which is movable on the guide rail (24) of the carriage (26) of the first transfer means, said slide (28) being pushed step by step to the exit of the buffer storage zone (16) by the carriage (26) of the first transfer means to clear said buffer storage zone (16) and being returned by the carriage (26) to the entry of the buffer storage zone when cleared.

15. Machine according to claim 14, **characterised in that** the slide (28) and carriage (26) are rigidly connected to each other by means of magnetic attraction when the carriage (26) is conveyed to the entry of the buffer storage zone (16), an abutment being provided at the entry of said zone to hold the slide (28) and release same from the carriage (26) when said carriage is returned to the entry of the cassette housing.

## Patentansprüche

1. Kassette zur Versorgung mit Reaktionsküvetten für ein automatisches Analysegerät, insbesondere zur Bestimmung der Änderungszeiten des physikalischen Zustands eines Mediums, **dadurch gekennzeichnet, dass** sie umfasst:
- zwei parallele Seitenwände (42), die fest miteinander zusammengebaut sind, und umfassend Tragmittel (46) für Reaktionsküvettenreihen (48),
- eine geschlossene Endseite, die durch Ränder (44) der Seitenwände (42) geformt wird und den Austritt der Küvetten aus der Kassette verhindern,
- und eine offene Endseite, die entgegengesetzt zur geschlossenen Endseite ist und mit einem gleitenden Verschluss (58) versehen ist, der zwischen einer Auslassposition der Reaktionsküvetten und einer Rückhalteposition der Reaktionsküvetten verschiebbar ist.

2. Kassette nach Anspruch 1, **dadurch gekennzeichnet, dass** die Tragmittel für Küvettenreihen (48) Rippen (46) umfassen, die vorspringend auf den Innenseiten der Seitenwände (42) geformt sind, und auf welchen die Seitenränder (50) der Küvetten (48) ruhen, und **dadurch**, dass die Innenseiten der Seitenwände (42) Flügel (52) umfassen, die parallel zu den Tragrippen (46) für Küvetten liegen und oberhalb dieser verlaufen, um die offenen oberen Enden der von diesen Rippen getragenen Küvetten (48) mindestens partiell zu schließen und den Austritt von Kugeln (54), die in den Küvetten enthalten sind, zu verhindern.

3. Kassette nach einem der vorherigen Ansprüche, **dadurch gekennzeichnet, dass** sie mindestens einen Ausstoßer umfasst, der durch ein Querplättchen (64) geformt wird, das zwischen den Seitenwänden (42) verläuft und auf den Trag- oder Führungsmitteln (46, 52) für Küvetten in Translation geführt wird, wobei dieses Plättchen seitliche Lappen oder Lappen (70) umfasst, die durch Schlitze der Seitenwände (42) der Kassette verlaufen und Mittel zum Antrieb des Plättchens von einem Ende der Kassette zum anderen formen, um alle Küvettenreihen (48) gleichzeitig zum offenen Ende der Kassette hin zu verschieben.

4. Kassette nach einem der vorherigen Ansprüche, **dadurch gekennzeichnet, dass** das geschlossene Ende der Kassette einen Handhabungsgriff (56) umfasst.

5. Kassette nach einem der vorherigen Ansprüche, **dadurch gekennzeichnet, dass** sie aus einem Metall oder aus einem spritzgegossenen oder warmgeformten Kunststoff hergestellt ist.

6. Automatisches Analysegerät, insbesondere zur Bestimmung der Änderungszeiten des physikalischen Zustands eines Mediums, umfassend Mittel zur Versorgung mit Reaktionsküvetten (48), **dadurch gekennzeichnet, dass** diese Versorgungsmittel umfassen:
- Mittel (10, 20, 22), die eine Aufnahme zur Unterbringung einer Kassette (14) nach einem der vorherigen Ansprüche formen,
- Mittel, die an einem Ende dieser Aufnahme vorgesehen sind und eine Pufferspeicherzone (16) für Reaktionsküvetten formen,
- erste Transfermittel (26, 32, 36, 76), um die Küvetten (48) der Kassette (14), die in der Aufnahme angeordnet ist, bis in die Pufferspeicherzone (16) hinein zu verschieben,
- und zweite Mittel (18, 28, 30), um die Küvetten aus der Pufferspeicherzone (16) bis zu einem Einlaufpunkt der Küvetten in das Analysegerät zu verschieben.

7. Gerät nach Anspruch 6, **dadurch gekennzeichnet, dass** die Antriebsmittel des oder der Ausstoßer(s) (64) in der Kassette (14) einen Wagen (26) umfassen, der durch Motormittel (32, 36) auf einer Längsführungsschiene (24) verschoben wird, wobei dieser Wagen mindestens eine zylindrische Querstange (76) umfasst, die entlang der Küvettenreihen senkrecht zu diesen Reihen verläuft und Finger (74) trägt, die dazu bestimmt sind, mit den seitlichen Lappen (70) des oder der Ausstoßer(s) (64) zusammenzuwirken, um sie von einem Ende der Küvettenreihen zum anderen zu verschieben und die Küvetten (48) aus der Kassette (14) austreten zu lassen.

8. Gerät nach Anspruch 7, **dadurch gekennzeichnet, dass** die oder jede zylindrische Stange (76) zwischen einer Arbeitsposition, in welcher die Finger (74) mit den seitlichen Lappen (70) des oder der Ausstoßer(s) (64) in Eingriff stehen, und einer Ruheposition, in welcher die Finger (74) von den seitlichen Lappen (70) des oder der Ausstoßer(s) (64) beabstandet sind, um ihre Achse drehbar auf dem Wagen (26) montiert ist.

9. Gerät nach Anspruch 7 oder 8, **dadurch gekennzeichnet, dass** die oder jede zylindrische Stange (76) Nocken trägt, die mit festen Anschlägen der Aufnahme der Kassette (14) zusammenwirken, um die Stange (76) aus einer der Positionen in die andere zu drehen und umgekehrt.

10. Gerät nach einem der Ansprüche 7 bis 9, **dadurch gekennzeichnet, dass** die Antriebsmittel des Wagens (26) einen Riemen (32) umfassen, der auf Riemenscheiben (34) geführt wird, wovon eine von Motormitteln (36) gedreht wird.

11. Gerät nach einem der Ansprüche 6 bis 10, **dadurch gekennzeichnet, dass** die Verschlussplatte (58) des Ausgangs der Kassette (14) durch ein bewegliches Organ (80), das am Eingang der Pufferspeicherzone (16) montiert ist und durch den Wagen (26) der ersten Transfermittel betätigt wird, zwischen ihren zwei Positionen bewegt wird, wobei diese Verschlussplatte (58) in ihrer Auslassposition der Küvetten (48) mit einem Anschlag zusammenwirkt, der in der Aufnahme der Kassette (14) vorgesehen ist, um die Kassette in ihrer Aufnahme zu verriegeln.

12. Gerät nach einem der Ansprüche 6 bis 11, **dadurch gekennzeichnet, dass** die Pufferspeicherzone (16) Küvettentragmittel umfasst, die in der Verlängerung der Küvettentragmittel (46) einer Kassette (14) verlaufen, die in der vorgenannten Aufnahme angeordnet ist, um Küvetten aus den in der Kassette enthaltenen Küvettenreihen aufzunehmen, und **dadurch**, dass die zweiten Transfermittel der Küvetten mindestens einen seitlichen Kamm (30) umfassen, dessen Zähne quer zwischen den Küvettentragmitteln der Pufferspeicherzone (16) verlaufen, um alle Küvetten aus dieser Zone gleichzeitig zu einem Förderband (86) zu schieben, das Küvetten am Ende der Küvettenreihen der Pufferspeicherzone (16) aufnimmt und sie nacheinander zu einem Einlaufpunkt der Küvetten im Analysegerät befördert.

13. Gerät nach einem der Ansprüche 6 bis 12, **dadurch gekennzeichnet, dass** in der Kassette (14) und in der Pufferspeicherzone (16) die Küvettenreihen (48) vertikal übereinanderliegen, und **dadurch**, dass das Förderband am Ausgang der Pufferspeicherzone (16) vertikal ist, das Band (86) des Förderers eine Reihe von Küvettenträgern (96) trägt, wobei die Zahl dieser Träger der Zahl der Küvettenreihen in der Pufferspeicherzone (16) entspricht.

14. Gerät nach Anspruch 12 oder 13, **dadurch gekennzeichnet, dass** der Kamm oder die Kämme (30) der zweiten Transfermittel von einem Schlitten (28) getragen werden, der auf der Führungsschiene (24) des Wagens (26) der ersten Transfermittel verschiebbar ist, wobei dieser Schlitten (28) vom Wagen (26) der ersten Transfermittel schrittweise zum Ausgang der Pufferspeicherzone (16) hin geschoben wird, um die Pufferspeicherzone (16) zu leeren, und von diesem Wagen (26) zum Eingang der Pufferspeicherzone zurückgeführt wird, wenn diese leer ist.

15. Gerät nach Anspruch 14, **dadurch gekennzeichnet, dass** der Schlitten (28) und der Wagen (26) durch magnetische Anziehungskraft verbunden sind, wenn der Wagen (26) zum Eingang der Pufferspeicherzone (16) geführt wird, wobei am Eingang dieser Zone ein Anschlag vorgesehen ist, um den Schlitten (28) zurückzuhalten und ihn vom Wagen (26) zu trennen, wenn der Wagen zum Eingang der Kassettenaufnahme zurückgeführt wird.
